# EUROPEAN PATENT SPECIFICATION

(11) **EP 2 589 411 B1**
(45) Date of publication and mention of the grant of the patent: **09.01.2019**
(21) Application number: 10854091.5
(22) Date of filing: 30.06.2010
(51) Int. Cl.: A61N 2/06, H01F 7/02

(54) **STRING-SHAPED MAGNET**
STRANGFÖRMIGER MAGNET
AIMANT FILIFORME

(43) Date of publication of application: 08.05.2013
(73) Proprietor: Komatsu, Katsumi, Osaka 558-0054 (JP)
(72) Inventor: NAKAMICHI, Naoki, Osaka-shi Osaka 542-0081 (JP)
(74) Representative: Bohnenberger, Johannes
(86) International application number: PCT/JP2010/061208
(87) International publication number: WO 2012/001794

(56) References cited:
- EP-A1- 1 980 170
- GB-A- 894 095
- JP-A- H10 165 211
- JP-A- S63 111 881
- JP-A- 2003 265 630
- JP-A- 2009 247 410
- JP-A- 2009 247 410
- JP-U- 3 085 787
- JP-U- 3 151 362
- JP-Y2- 5 045 315
- US-A1- 2008 072 622
- US-A1- 2008 146 864

## Description

### TECHNICAL FIELD

The present invention relates to a string-shaped magnet used as a magnetic treatment device and such.

### BACKGROUND ART

Conventionally, permanent magnets are used as magnetic treatment devices, and there has been proposed a neck-type magnetic treatment device in which a plurality of permanent magnets are disposed along an annular band (see Japanese Unexamined Patent Application Publication No. 2004-147859, for example). These permanent magnets are worn around a neck portion of a body so as to facilitate the flow of blood or to relax muscles by magnetism, thereby treating neck/shoulder stiffness.

A string shaped magnet according to the preamble of the appended independent claim is described in JPH10-165211.

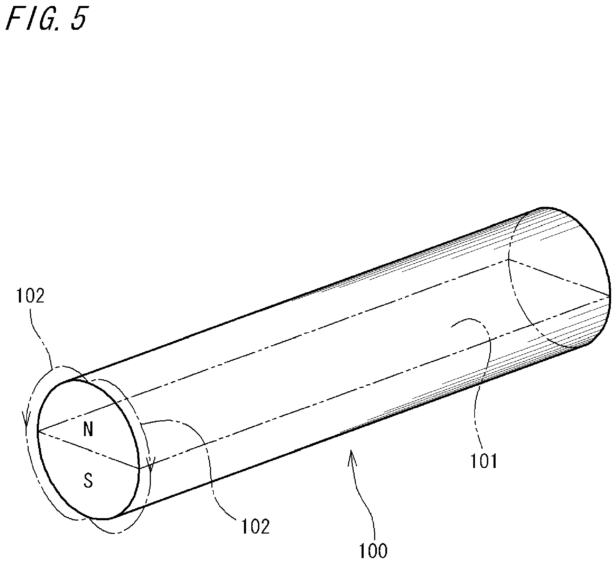

However, as a magnetic treatment device using such permanent magnets needs a magnetism capsule (housing) for storing a plurality of permanent magnets, there are problems of an increased cost and low flexibility in design. To address these problems, as illustrated in FIG. 5, there is known a string-shaped magnet in which a flexible strand body containing a magnetic powder is used as a material for a permanent magnet 100, and the strand body is magnetized such that a boundary surface 101 between magnetic polarities is continuously formed along its longitudinal direction.

However, in such a string-shaped magnet, as the north pole and the south pole are continuous along the longitudinal direction, magnetic lines 102 of this magnet are generated only in a small area along an outer peripheral surface of the string-shaped magnet. Therefore, if this string-shaped magnet is used as a magnetic treatment device, it is not possible to make the magnetism thoroughly penetrate into the body, and thus a new problem of reducing the treatment effect arises.

### DISCLOSURE OF INVENTION

The invention is defined as disclosed by the appended independent claim. A preferred embodiment is illustrated in the dependent claim.

The present invention has been made in view of the above circumstances, and an object of the present invention is to provide a string-shaped magnet capable of generating field lines across a wide area.

In order to achieve the above object, a string-shaped magnet according to the present invention includes: a flexible strand body made of synthetic resin and containing a magnetic powder; and a plurality of magnetic parts disposed at a predetermined pitch in a longitudinal direction of the strand body, wherein polarities of the magnetic parts that are adjacent to each other are opposite to each other.

According to the present invention, as the polarities of the magnetic parts that are adjacent in the longitudinal direction of the strand body are opposite to each other, it is possible to generate field lines between the adjacent magnetic parts across a wide area.

Further, it is preferable that the string-shaped magnet further include a pair of fasteners respectively disposed at both ends of the strand body in the longitudinal direction, the fasteners being engageable with and removable from each other.

In this case, the string-shaped magnet can be easily worn around the body, allowing the field lines to act effectively on a point of stiffness in the body.

### BRIEF DESCRIPTION OF DRAWINGS

FIG. 1 is a perspective view illustrating a string-shaped magnet.
FIG. 2 is a flowchart showing a manufacturing process of the string-shaped magnet.
FIG. 3 is a perspective view illustrating an application example of the string-shaped magnet in which the magnet is worn around a neck portion as a magnetic treatment device.
FIG. 4 is a perspective view illustrating fasteners of the string-shaped magnet.
FIG. 5 is a perspective view illustrating a conventional string-shaped magnet.

### DESCRIPTION OF EMBODIMENTS

Hereinafter, a preferred embodiment of the present invention will be described with reference to the accompanying drawings.

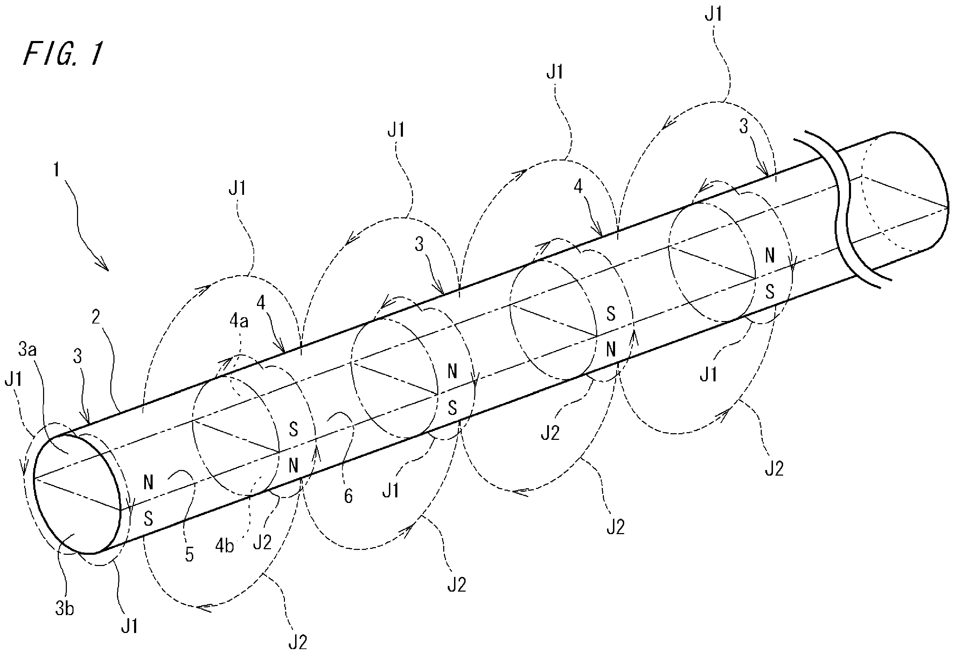

FIG. 1 is a perspective view illustrating a string-shaped magnet 1. The string-shaped magnet 1 is configured such that a strand body 2 made of synthetic resin includes a plurality of first magnetic parts 3 and second magnetic parts 4 arranged alternately in series along a longitudinal direction of the strand body 2.

The strand body 2 is a flexible and string-shaped body having a circular cross-section and containing a magnetic powder. Each first magnetic part 3 is magnetized such that a magnetic polar boundary surface 5 is formed planarly along the longitudinal direction (axial direction) of the strand body 2. The polar boundary surface 5 is formed so as to radially divide the first magnetic part 3 in half, and such that a semicircular columnar portion 3a on one side (upper side in FIG. 1) and a semicircular columnar portion 3b on the other side (lower side in FIG. 1) of the polar boundary surface 5 are magnetized to respectively constitute the north pole and the south pole.

Each second magnetic part 4 is magnetized such that a planar polar boundary surface 6 is formed along the longitudinal direction of the strand body 2 substantially in the same plane as the polar boundary surface 5. The polar boundary surface 6 is formed so as to radially divide the second magnetic part 4 in half, and each second magnetic part 4 is magnetized so as to have magnetic polarities opposite to those of an adjacent one of the first magnetic parts 3. Specifically, a semicircular columnar portion 4a on one side (upper side in FIG. 1) and a semicircular columnar portion 4a on the other side (lower side in FIG. 1) of the polar boundary surface 6 are magnetized to respectively constitute the south pole and the north pole.

With the above configuration, field lines J1 generated from the north pole of each first magnetic part 3 reach not only the south pole of this first magnetic part 3 but also the south pole of an adjacent one of the second magnetic parts 4 in the longitudinal direction. Further, field lines J2 generated from the north pole of the adjacent second magnetic part 4 reach not only the south pole of this second magnetic part 4 but also the south pole of the adjacent first magnetic parts 3 in the longitudinal direction. As illustrated in FIG. 1, the field lines J1 and J2 generated in the longitudinal direction are maximized at a central portion between adjacent ones of the magnetic parts 3 and 4 in the longitudinal direction.

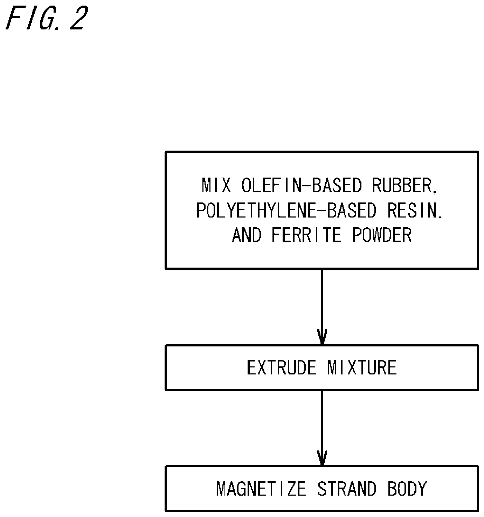

FIG. 2 is a flowchart showing one example of a manufacturing process of the string-shaped magnet 1. Referring to the chart, first, a flexible olefin-based rubber, an ethylene-based resin which is a thermoplastic synthetic resin, and a ferrite powder which is a magnetic powder are mixed by a mixing machine that is not shown in the drawings (mixing step). Examples of the olefin-based rubber include an ethylene-propylene copolymer rubber and an ethylene-butylene copolymer rubber. Further, the ethylene-based resin has good miscibility in the olefin-based rubber, and examples of this resin include a low-density polyethylene resin, a high-density polyethylene resin, a linear low-density polyethylene resin, and an ethylene-vinyl acetate copolymer resin.

Next, a mixture resulting from the mixing step is extruded by an extruder that is not shown in the drawings to be molded into the strand body 2 having a circular cross-section (molding step).

Then, the strand body 2 goes through a magnetizer (not shown in the drawings) to be sequentially magnetized. At this time, magnetizing the strand body 2 such that the north pole and the south pole are inverted at predetermined intervals provides the first magnetic parts 3 having the polar boundary surface 5 and the second magnetic parts 4 having the polar boundary surface 6 alternately in series along the longitudinal direction (magnetizing step). Thus, the flexible string-shaped magnet 1 is manufactured.

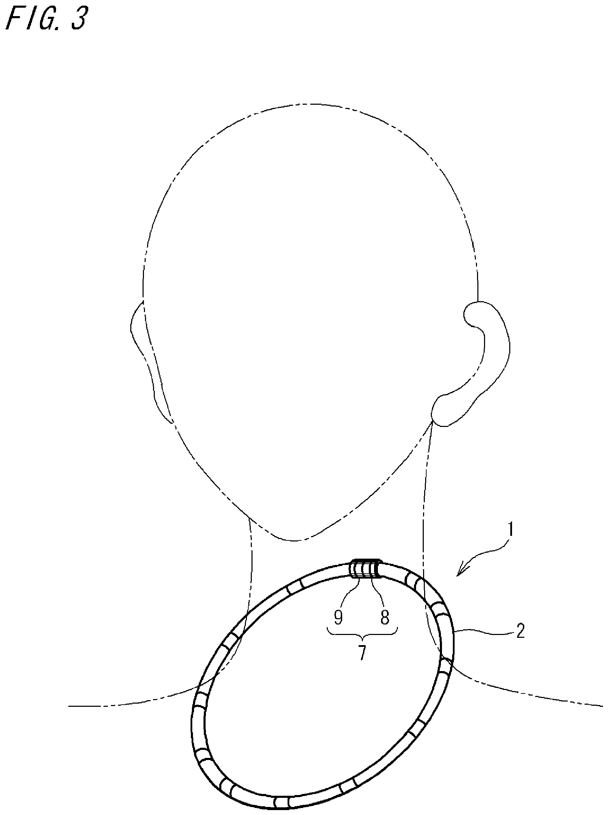

FIG. 3 is a perspective view illustrating an application example of the string-shaped magnet 1, in which the magnet is worn annularly around a neck portion of a body as a magnetic treatment device for treating neck/shoulder stiffness. The string-shaped magnet 1 is provided with a fastening unit 7 on both ends of the strand body 2.

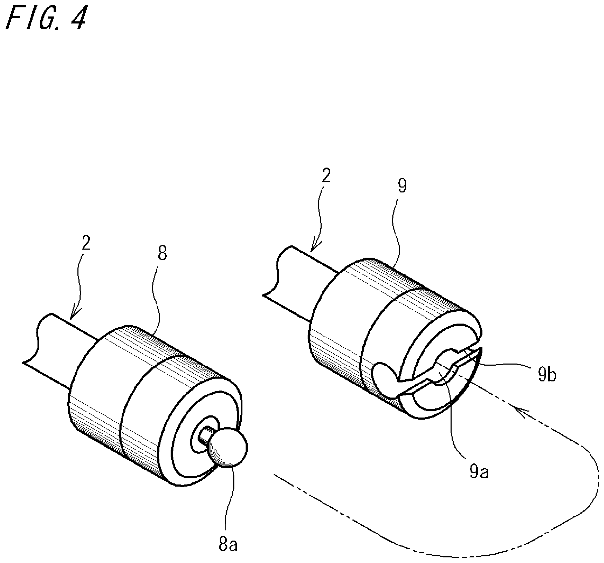

As illustrated in FIG. 4, the fastening unit 7 includes a pair of fasteners having a male fastener 8 and a female fastener 9 that are engageable with and removable from each other. The male fastener 8 has a ball-shaped projection 8a at a tip thereof, and the female fastener 9 has a depressed portion 9a at a tip thereof, the depressed portion 9a rotatably holding the projection 8a. The tip of the female fastener 9 is divided by a slit 9b in half in a manner elastically expansible.

The both ends of the strand body 2 can be connected by depressing the projection 8a of the male fastener 8 into the depressed portion 9a of the female fastener 9, and disconnected either by pulling the male fastener 8 and the female fastener 9 in opposite directions from each other or by mountain-folding or valley-folding at a butting plane between the male fastener 8 and the female fastener 9.

Further, the male fastener 8 and the female fastener 9 when connected are rotatable in a circumferential direction centering the projection 8a. This allows to easily correct twisting of the strand body 2 that can easily occur when connecting the fasteners while the fasteners are being connected.

As illustrated in FIG. 3, the string-shaped magnet 1 is worn around the neck portion of the body, by fitting the male fastener 8 and the female fastener 9 provided for the both ends of the strand body 2 with each other while the string-shaped magnet 1 is turned around the neck portion. By causing magnetism of the first magnetic parts 3 and the second magnetic parts 4 to act effectively on a point of neck/shoulder stiffness in this state, it is possible to facilitate the flow of blood or to relax muscles, as well as to improve its treatment effect. In addition, the field lines J1 and J2 are generated across a boundary between the first magnetic part 3 and the second magnetic part 4 that are adjacent, and therefore the field lines J1 and J2 can be generated across a wide area outside an outer peripheral surface of the string-shaped magnet 1. Therefore, it is possible to make the magnetism thoroughly penetrate into and across a wide area of the body. With this, it is possible to prevent reduction of the treatment effect of the string-shaped magnet 1 according to the present invention when the string-shaped magnet 1 is used as a magnetic treatment device.

It should be noted that the present invention is not limited to the embodiment described above. For example, while the strand body 2 has a circular cross-section in the above embodiment, the strand body 2 may have a polygonal cross-section or an elliptical cross-section.

Further, in the above embodiment, the first magnetic part 3 and the second magnetic part 4 are disposed without any space therebetween in the longitudinal direction. According to the present invention a non-magnetic part of a predetermined width may be disposed between the first magnetic part 3 and the second magnetic part 4.

Moreover, the string-shaped magnet 1 according to the present invention can be used in an application of a magnetic treatment device that is worn around such as the wrist or the ankle, in addition to the application of the magnetic treatment device that is worn around the neck portion.

Furthermore, the string-shaped magnet 1 according to the present invention can be used in various applications other than the magnetic treatment device.

## Claims

1. A string-shaped magnet (1) comprising:
an extruded flexible strand body (2) made of synthetic resin and containing a magnetic powder; and
a plurality of magnetic parts (3, 4) disposed at a predetermined pitch in a longitudinal direction of the extruded flexible strand body, wherein
polarities of the magnetic parts (3, 4) that are subsequent to each other along the longitudinal direction are opposite to each other; and **characterized by** the extruded flexible strand body (2) including a non-magnetic part between the subsequent magnetic parts (3, 4),
the non-magnetic part is integrally formed with the magnetic parts (3, 4) during extrusion of the strand body and magnetization of magnetic powder to form the magnetic parts (3, 4) so that the non-magnetic part have exactly the same external cross-sectional shape as the subsequent magnetic parts (3, 4).

2. The string-shaped magnet (1) according to claim 1, further comprising: a pair of fasteners (8, 9) respectively disposed at both ends of the extruded flexible strand body (2) in the longitudinal direction, the fasteners (8, 9) being engageable with and removable from each other.

## Patentansprüche

1. Strangförmiger Magnet (1), umfassend:
einen extrudierten flexiblen Strangkörper (2), der aus Kunstharz hergestellt ist und ein Magnetpulver enthält; und
mehrere magnetische Teile (3, 4), die in einem vorbestimmten Abstand in einer Längsrichtung des extrudierten flexiblen Strangkörpers angeordnet sind, wobei Polaritäten der magnetischen Teile (3, 4), die entlang der Längsrichtung aufeinanderfolgen, einander entgegengesetzt sind; und
**dadurch gekennzeichnet, dass**
der extrudierte flexible Strangkörper (2) einen nicht-magnetischen Teil zwischen den aufeinanderfolgenden magnetischen Teilen (3, 4) umfasst,
der nicht-magnetische Teil mit den magnetischen Teilen (3, 4) während der Extrusion des Strangkörpers und Magnetisierung von Magnetpulver zum Bilden der magnetischen Teile (3, 4) einstückig ausgebildet wird, sodass die nicht-magnetischen Teile genau dieselbe äußere Querschnittsform aufweisen wie die aufeinanderfolgenden magnetischen Teile (3, 4).

2. Strangförmiger Magnet (1) nach Anspruch 1, ferner umfassend:
ein Paar von Verschlüssen (8, 9), die jeweils an beiden Enden des extrudierten flexiblen Strangkörpers (2) in der Längsrichtung angeordnet sind, wobei die Verschlüsse (8, 9) in gegenseitigen Eingriff bringbar und voneinander lösbar sind.

## Revendications

1. Aimant filiforme (1) comprenant :
un corps filaire flexible extrudé (2) constitué de résine synthétique et contenant une poudre magnétique ; et
une pluralité de parties magnétiques (3, 4) disposées selon un pas prédéterminé en direction longitudinale du corps filaire flexible extrudé, dans lequel les polarités des parties magnétiques (3, 4) subséquentes en direction longitudinale sont opposées entre elles ; et
**caractérisé en ce que**
le corps filaire flexible extrudé (2) comprend une partie non magnétique entre les parties magnétiques subséquentes (3, 4),
la partie non magnétique étant formée intégralement avec les parties magnétiques (3, 4) durant l'extrusion du corps filaire et la magnétisation de la poudre magnétique pour former les parties magnétiques (3, 4) de telle sorte que la partie non magnétique présente exactement la même forme externe en section transversale que les parties magnétiques subséquentes (3, 4).

2. Aimant filiforme (1) selon la revendication 1, comprenant en outre :
une paire de fermoirs (8, 9) disposés respectivement aux deux extrémités du corps filaire flexible extrudé (2) en direction longitudinale, les fermoirs (8, 9) pouvant être mutuellement engagés et détachés.
